# EUROPEAN PATENT APPLICATION

(11) **EP 2 962 694 A1**
(43) Date of publication of application: **06.01.2016**
(21) Application number: 14002295.5
(22) Date of filing: 04.07.2014
(51) Int. Cl.: A61K 38/01, C07K 14/47

(54) **Novel polypeptides and their use**

(71) Applicant: Université de Lausanne, 1011 Lausanne (CH)
(72) Inventor: Petrova, Tatiana, 1066 Epalinges (CH); Sabine, Amélie, 1005 Lausanne (CH)
(74) Representative: Helbig, Christian

(57) **Abstract**

The invention relates to novel polypeptides, nucleic acids encoding same, vectors comprising same, host cells and their use.

SEQUENCE LISTING

<110>  University of Lausanne
       University of Lausanne

<120>  Novel Polypeptides and Their use

<130>  UL20223EP

<160>  7

<170>  PatentIn version 3.5

<210>  1
<211>  3066
<212>  DNA
<213>  human

<400>  1
ttccttttat gcgaacacaa gtatatgagg ggttgtgtaa aaattatttt tctcttgctg      60

tactacaaag agatagaatc aaactgcttt ttttcgacat actggttttt ctttctgttt     120

ttcttctctt tcttctattt cttgtggata ttatggctaa taacacaaca agtttaggga     180

gtccatggcc agaaaacttt tgggaggacc ttatcatgtc cttcactgta tccatggcaa     240

tcgggctggt acttggagga tttatttggg ctgtgttcat ttgtctgtct cgaagaagaa     300

gagccagtgc tcccatctca cagtggagtt caagcaggag atctaggtct tcttacaccc     360

acggcctcaa cagaactgga ttttaccgcc acagtggctg tgaacgtcga agcaacctca     420

gcctggccag tctcaccttc cagcgacaag cttccctgga acaagcaaat tcctttccaa     480

gaaaatcaag tttcagagct tctactttcc atccctttct gcaatgtcca ccacttcctg     540

tggaaactga gagtcagctg gtgactctcc cttcttccaa tatctctccc accatcagca     600

cttcccacag tctgagccgt cctgactact ggtccagtaa cagtcttcga gtgggccttt     660

caacaccgcc cccacctgcc tatgagtcca tcatcaaggc attcccagat tcctgagtag     720

ggtggctttt ggtttttgtt tctttcttgt cttgtctttt attgaaagga aatcaaaaat     780

aggctaaaca gaattttgag ggcatggccc aaataactca tgagttccaa gttgaaacat     840

ggttgtgcaa gttggacatt acaatgtaaa acacattttc ttcaaacacg ttttcccttt     900

tgtttcaaaa aatgtaatat tttcccccaa gcgttttata tttatgtatt ttgtattcaa     960

tgtgaggctt attaaaaata gtgattctaa tgtaagaatc agctaagatg cattatatat    1020

attttaatta aaattaaaac ttcagatatt tgtggattac aatcctcatt tacttccaat    1080

gtgactaaaa agagaaaaaa aatcactgtg tcactttaaa gaaaaatctt ctaagggatt    1140

tggattttac tttctttaga atgacaagtg aatcatattg acattttaca atcttagatt    1200

tttctttttt tttcttttga gacagggtct tgatccgtcg cccaggcggg agttgcagta    1260

gcatgatcag gactcactgc agcctctatc tcccaggctc aagtaatcct cccatcttag    1320

tgccccaagt agctgggact acaggggtgc actaccacac cgggttgaat ttttttttaa 1380

ttttagtaga gatgaagtgt cactatgtta ccaaggctgg tctcaaactc ctaaactcag 1440

atgatcctcc tgcctcggcc tcccaaagtg ctggaattag cctggccaat cttggatttt 1500

taatggaata tgtgggcaca aaatgacaga acataggaca ttctaaagtt ccttgatttg 1560

atcattataa gaagtgtggg actcaagcac aggaaactga actcttttgg tgtcattgga 1620

tgtttcattt ttgacactaa ttttttctgg acaaactctt tatgtgtttt tcccaagaat 1680

agttatctac ttcctggagg caaaatcctt ggatttacta acatgatgat ttaccttttc 1740

ttcaccgttg tcgttacatt gttagaaaag caacaggaaa aaatccaatt catttgacct 1800

aaaaacaagc ctcaagttta aaaccaagct cacgtttttc ttaagggaaa aattttcttt 1860

cttaaactta catctagcaa cttggaaagc actttctctg gggatcttct tttgtaactt 1920

tgcagacaaa taagtatgag tcactgggga gagagtttgt tattgaaata gatgttgccc 1980

atgaagaatt ctccttcctg gattgactct taatcatcag gcatcattcc tggtttgctt 2040

ctctacgaat ctcaattcca acttctctgc agagtctgta cagtgattaa gccatgccag 2100

atggtctttg gtgcacacag ttatttaaga atccacttcc acaggtggct gcccttgtaa 2160

ggaagaatgc atccctaaat gtggccacca gagagttcca gtgggcagat gtctgtggct 2220

gcccttctca tttaaggaca tgagttcact ggagtattac tcaaaaagtc tgtggttcat 2280

ttccagtatt gtgaatattt agtttatgtg gccgtttctt tgtttctttg aacagtggga 2340

ttttcagtga aaaagtaccc tctttttcat ttcctattgc agtggtcaca gctaatagtg 2400

tctgaacatg gttcaagaat aagagattcc atgtagcatt ttctttatta ttttcatttc 2460

ccttatatta tccatcattc cttaaggaca attattctta ataatgctta tagaaaatgt 2520

tctctaatta aacatgccaa aaggaaaaag taagagaaag agggagcaag aagaaaatgg 2580

aagaaaaagg gaaaaaagct aaccggataa ccaatttgtt ataagttggt tttcaacaaa 2640

gaaatttagc agccaagtaa ggtttcaagg gaatattaac ttggtatcag ggctactttt 2700

tttttttttt ttttacttgc atgtcatcct taatgtctaa catgaaaaat cagcaaagag 2760

tatggttttt atcaagaatt tgtgttggga gtaaaaactg ctttatagct cccaaattag 2820

gaagagaaga gcagaaatcc tctggggcat ttaaccatct ggcagaattg ttgctgcacc 2880

cttatcccag ttataagaca gtcaaaatga ctatttccta aatattgtga gtgtatgaaa 2940

tgtgaaatta aagcaaaaac tggagacttt taatgtattt ctttaatttg aaatgttttg 3000

tggattgtga aataaaaata aatttatgtc aagttttatt caaaaaaaaa aaaaaaaaaa 3060

aaaaaa 3066

<210> 2
<211> 235

<212>  PRT
<213>  human

<400>  2

Met Arg Thr Gln Val Tyr Glu Gly Leu Cys Lys Asn Tyr Phe Ser Leu
1               5                   10                  15

Ala Val Leu Gln Arg Asp Arg Ile Lys Leu Leu Phe Phe Asp Ile Leu
            20                  25                  30

Val Phe Leu Ser Val Phe Leu Leu Phe Leu Leu Phe Leu Val Asp Ile
        35                  40                  45

Met Ala Asn Asn Thr Thr Ser Leu Gly Ser Pro Trp Pro Glu Asn Phe
    50                  55                  60

Trp Glu Asp Leu Ile Met Ser Phe Thr Val Ser Met Ala Ile Gly Leu
65                  70                  75                  80

Val Leu Gly Gly Phe Ile Trp Ala Val Phe Ile Cys Leu Ser Arg Arg
                85                  90                  95

Arg Arg Ala Ser Ala Pro Ile Ser Gln Trp Ser Ser Ser Arg Arg Ser
            100                 105                 110

Arg Ser Ser Tyr Thr His Gly Leu Asn Arg Thr Gly Phe Tyr Arg His
        115                 120                 125

Ser Gly Cys Glu Arg Arg Ser Asn Leu Ser Leu Ala Ser Leu Thr Phe
    130                 135                 140

Gln Arg Gln Ala Ser Leu Glu Gln Ala Asn Ser Phe Pro Arg Lys Ser
145                 150                 155                 160

Ser Phe Arg Ala Ser Thr Phe His Pro Phe Leu Gln Cys Pro Pro Leu
                165                 170                 175

Pro Val Glu Thr Glu Ser Gln Leu Val Thr Leu Pro Ser Ser Asn Ile
            180                 185                 190

Ser Pro Thr Ile Ser Thr Ser His Ser Leu Ser Arg Pro Asp Tyr Trp
        195                 200                 205

Ser Ser Asn Ser Leu Arg Val Gly Leu Ser Thr Pro Pro Pro Pro Ala
    210                 215                 220

Tyr Glu Ser Ile Ile Lys Ala Phe Pro Asp Ser
225                 230                 235

<210>  3
<211>  187
<212>  PRT
<213>  human

<400>  3

Met Ala Asn Asn Thr Thr Ser Leu Gly Ser Pro Trp Pro Glu Asn Phe
1               5                   10                  15

Trp Glu Asp Leu Ile Met Ser Phe Thr Val Ser Met Ala Ile Gly Leu
            20                  25                  30

Val Leu Gly Gly Phe Ile Trp Ala Val Phe Ile Cys Leu Ser Arg Arg
        35                  40                  45

Arg Arg Ala Ser Ala Pro Ile Ser Gln Trp Ser Ser Ser Arg Arg Ser
    50                  55                  60

Arg Ser Ser Tyr Thr His Gly Leu Asn Arg Thr Gly Phe Tyr Arg His
65                  70                  75                  80

Ser Gly Cys Glu Arg Arg Ser Asn Leu Ser Leu Ala Ser Leu Thr Phe
                85                  90                  95

Gln Arg Gln Ala Ser Leu Glu Gln Ala Asn Ser Phe Pro Arg Lys Ser
            100                 105                 110

Ser Phe Arg Ala Ser Thr Phe His Pro Phe Leu Gln Cys Pro Pro Leu
        115                 120                 125

Pro Val Glu Thr Glu Ser Gln Leu Val Thr Leu Pro Ser Ser Asn Ile
    130                 135                 140

Ser Pro Thr Ile Ser Thr Ser His Ser Leu Ser Arg Pro Asp Tyr Trp
145                 150                 155                 160

Ser Ser Asn Ser Leu Arg Val Gly Leu Ser Thr Pro Pro Pro Pro Ala
                165                 170                 175

Tyr Glu Ser Ile Ile Lys Ala Phe Pro Asp Ser
            180                 185


<210>  4
<211>  560
<212>  DNA
<213>  human

<400>  4

atggctaata acacaacaag tttagggagt ccatggccag aaaacttttg ggaggacctt 60

atcatgtcct tcactgtatc catggcaatc gggctggtac ttggaggatt tatttgggct 120

gtgttcattt gtctgtctcg aagaagaaga gccagtgctc ccatctcaca gtggagttca 180

agcaggagat ctaggtcttc ttacacccac agcctcaaca gaactggatt ttaccgccac 240

agtggctgtg aacgtcgaag caacctcagc ctggccagtc tcaccttcca gcgacaagct 300

tccctggaac aagcaaattc ctttccaaga aaatcaagtt tcagagcttc tactttccat 360

ccctttctgc aatgtccacc acttcctgtg gaaactgaga gtcagctggt gactctccct 420

tcttccaata tctctcccac catcagcact tcccacagtc tgagccgtcc tgactactgg 480

tccagtaaca gtcttcgagt gggcctttca acaccgcccc cacctgcctt tgagtccatc 540

atcaaggcat tcccagattc 560

<210> 5
<211> 187
<212> PRT
<213> human

<400> 5

Met Ala Asn Asn Thr Thr Ser Leu Gly Ser Pro Trp Pro Glu Asn Phe
1               5                   10                  15

Trp Glu Asp Leu Ile Met Ser Phe Thr Val Ser Met Ala Ile Gly Leu
            20                  25                  30

Val Leu Gly Gly Phe Ile Trp Ala Val Phe Ile Cys Leu Ser Arg Arg
        35                  40                  45

Arg Arg Ala Ser Ala Pro Ile Ser Gln Trp Ser Ser Ser Arg Arg Ser
    50                  55                  60

Arg Ser Ser Tyr Thr His Ser Leu Asn Arg Thr Gly Phe Tyr Arg His
65                  70                  75                  80

Ser Gly Cys Glu Arg Arg Ser Asn Leu Ser Leu Ala Ser Leu Thr Phe
                85                  90                  95

Gln Arg Gln Ala Ser Leu Glu Gln Ala Asn Ser Phe Pro Arg Lys Ser
            100                 105                 110

Ser Phe Arg Ala Ser Thr Phe His Pro Phe Leu Gln Cys Pro Pro Leu
        115                 120                 125

Pro Val Glu Thr Glu Ser Gln Leu Val Thr Leu Pro Ser Ser Asn Ile
    130                 135                 140

Ser Pro Thr Ile Ser Thr Ser His Ser Leu Ser Arg Pro Asp Tyr Trp
145                 150                 155                 160

Ser Ser Asn Ser Leu Arg Val Gly Leu Ser Thr Pro Pro Pro Pro Ala
                165                 170                 175

Phe Glu Ser Ile Ile Lys Ala Phe Pro Asp Ser
            180                 185

<210>  6
<211>  145
<212>  PRT
<213>  human

<400>  6

Ile Cys Leu Ser Arg Arg Arg Arg Ala Ser Ala Pro Ile Ser Gln Trp
1               5                   10                  15

Ser Ser Ser Arg Arg Ser Arg Ser Ser Tyr Thr His Gly Leu Asn Arg
            20                  25                  30

Thr Gly Phe Tyr Arg His Ser Gly Cys Glu Arg Arg Ser Asn Leu Ser
        35                  40                  45

Leu Ala Ser Leu Thr Phe Gln Arg Gln Ala Ser Leu Glu Gln Ala Asn
    50                  55                  60

Ser Phe Pro Arg Lys Ser Ser Phe Arg Ala Ser Thr Phe His Pro Phe
65                  70                  75                  80

Leu Gln Cys Pro Pro Leu Pro Val Glu Thr Glu Ser Gln Leu Val Thr
                85                  90                  95

Leu Pro Ser Ser Asn Ile Ser Pro Thr Ile Ser Thr Ser His Ser Leu
            100                 105                 110

Ser Arg Pro Asp Tyr Trp Ser Ser Asn Ser Leu Arg Val Gly Leu Ser
        115                 120                 125

Thr Pro Pro Pro Pro Ala Tyr Glu Ser Ile Ile Lys Ala Phe Pro Asp
    130                 135                 140

Ser
145

<210>  7
<211>  144
<212>  PRT

<213>  human

<400>  7

Cys Leu Ser Arg Arg Arg Arg Ala Ser Ala Pro Ile Ser Gln Trp Ser
1               5                   10                  15

Ser Ser Arg Arg Ser Arg Ser Ser Tyr Thr His Ser Leu Asn Arg Thr
            20                  25                  30

Gly Phe Tyr Arg His Ser Gly Cys Glu Arg Arg Ser Asn Leu Ser Leu
        35                  40                  45

Ala Ser Leu Thr Phe Gln Arg Gln Ala Ser Leu Glu Gln Ala Asn Ser
    50                  55                  60

Phe Pro Arg Lys Ser Ser Phe Arg Ala Ser Thr Phe His Pro Phe Leu
65                  70                  75                  80

Gln Cys Pro Pro Leu Pro Val Glu Thr Glu Ser Gln Leu Val Thr Leu
                85                  90                  95

Pro Ser Ser Asn Ile Ser Pro Thr Ile Ser Thr Ser His Ser Leu Ser
            100                 105                 110

Arg Pro Asp Tyr Trp Ser Ser Asn Ser Leu Arg Val Gly Leu Ser Thr
        115                 120                 125

Pro Pro Pro Pro Ala Phe Glu Ser Ile Ile Lys Ala Phe Pro Asp Ser
    130                 135                 140

## Description

The invention relates to novel polypeptides, nucleic acids encoding same, vectors comprising same, host cells and their use.

### Background of the Invention

Cancer is a leading cause of mortality worldwide. During the last decade a new class of treatments has emerged, the so-called "anti-angiogenic" drugs that target the tumor vascular network, and these treatments are based upon the inhibition of vascular endothelial growth factor (VEGF) signaling.

However, the use of anti-VEGF drugs during the past few years has raised several fundamental questions. Despite the promising results obtained in animal models, results of anti-VEGF therapeutics have been disappointing in humans as they do not cure cancer patients even if they prolong life by several months.

Patients can become resistant to these treatments and the mechanisms implicated are still largely unknown. Moreover, these drugs are associated with relatively rare but severe side effects.

Thus, there is still a great need for fundamental research to identify new signaling pathways for targeting tumor vasculature in addition to or independently of VEGF.

Research in this context revealed knowledge of a number of factors which are involved in physiological processes and which may have a significance in diseases.

In Leukemia (2012) 26, 177-179; doi:10.1038/Ieu.2011.188; published online 29 July 2011 (by K Liddiard, AK Burnett, RL Darley1 and A Tonks) it was discussed that RUNX1-ETO deregulates the proliferation and growth factor responsiveness of human hematopoietic progenitor cells downstream of the myeloid transcription factor, MYCT1.

Fu S, Guo Y, Chen H, Xu Z-M, Qiu G-B, et al. (2011) describe "MYCT1-TV, A Novel MYCT1 Transcript, Is Regulated by c-Myc and May Participate in Laryngeal Carcinogenesis" in PLoS ONE 6(10): e25648. doi:10.1371/journal.pone.0025648.

Xiaoying Yin*, Linnette Grove*, Kenneth Rogulski‡, and Edward V. Prochownik (THE JOURNAL OF BIOLOGICAL CHEMISTRY, Vol. 277, No. 22, Issue of May 31, pp. 19998-20010, 2002) published an article relating to Myc Target in Myeloid Cells-1, a Novel c-Myc Target, Recapitulates Multiple c-Myc Phenotypes.

Kenneth R. Rogulski*, Debra E. Cohen*, David L. Corcoran†‡, Panayiotis V. Benos†‡, and Edward V. Prochownik (18968-18973, PNAS , December 27, 2005 , vol. 102 , no. 52) describe the deregulation of common genes by c-Myc and its direct target, MT-MC1.

Guang-Bin Qiu, Li-Guo Gong, Dong-Mei Hao, Zhi-Hong Zhen, Kai-Lai Sun (World J Gastroenterol 2003;9(10):2160-2163) describe the expression of MTLC gene in gastric carcinoma.

Krisiti Rothermund, Kenneth Rogulski, Elaine Fernandes, Amy Whiting4 John Sedivy, Lixia Pu, and Edward V. Prochownik (Cancer Res 2005; 65: (6). March 15, 2005) describe C-Myc-Independent Restoration of Multiple Phenotypes by Two C-Myc Target Genes with Overlapping Functions.

Min Yang1,2†, Wei Li1,3†, Yi-Ying Liu1, Shuang Fu1, Guang-Bin Qiu4, Kai-Lai Sun1 and Wei-Neng Fu1 (BMC Cancer 2012, 12:219) research the promoter hypermethylation-induced transcriptional down-regulation of the gene MYCT1 in laryngeal squamous cell carcinoma.

None of the above cited research articles describes or suggests the invention of the current application, nor do they include any disclosure that would motivate the skilled person to embark on the route the current inventors went to develop their solution for the problem underlying the present application.

Equally important is the diagnosis and the availability of diagnostic tools in the context of cancer and other diseases like retinal vascular disease etc.

A number of endothelial-specific genes have been identified and characterized, and their implications are acknowledged for medical and diagnostic applications in research.

Hence it is one object underlying the current application to provide novel factors, preferably polypeptides, useful in medical applications and particular in cancer applications as well as in diagnostics. Another object is to provide for means superior to known disease treatments, their prevention or representing an alternative to the known treatments. Another object is to provide for means useful in diagnosing such diseases.

### Summary of the Invention

In one aspect the invention relates to novel polypeptides and nucleic acids encoding same.

In another aspect the invention relates to compositions comprising the novel polypeptides.

In yet another aspect the invention relates to the medical or diagnostic use of said polypeptides and compositions.

In yet another aspect the invention relates to the novel polypeptides and/or compositions for use in the prevention or treatment of cancer, a retinal vascular disease, wound healing, ischemia, myocardial infarction, or atherosclerosis.

In another aspect the invention relates to diagnostic methods using the novel polypeptides of the invention.

### Brief Description of the Figures

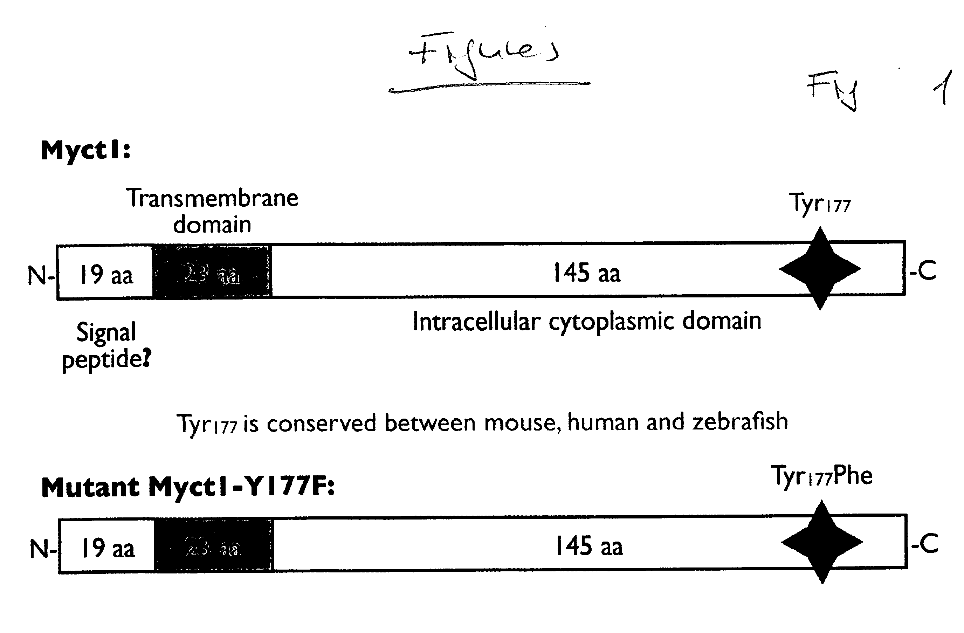

Fig. 1 depicts a polypeptide according to the invention exhibiting in position 177 a phenylalanine residue in the short form of MYCT1

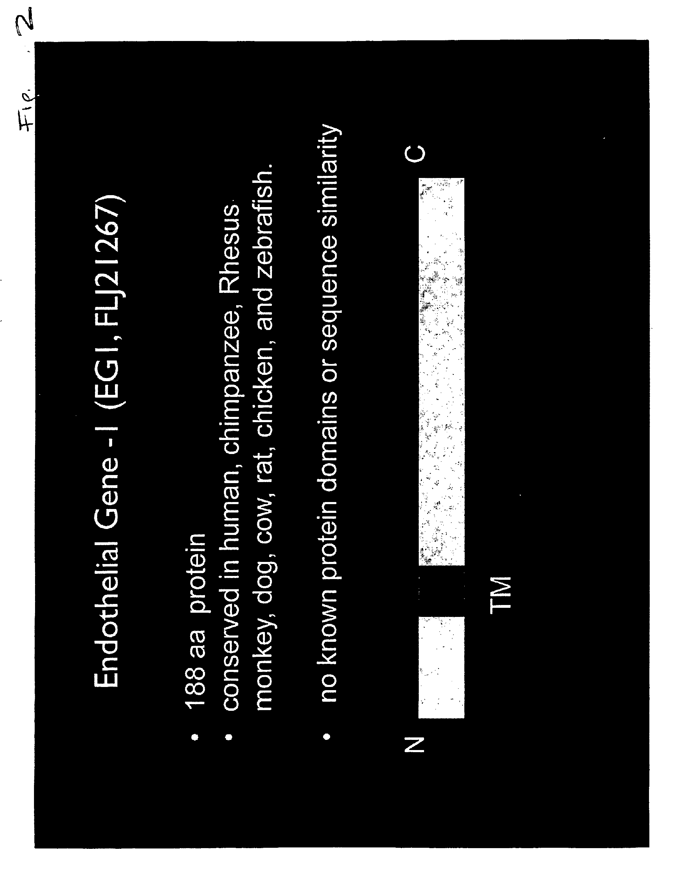

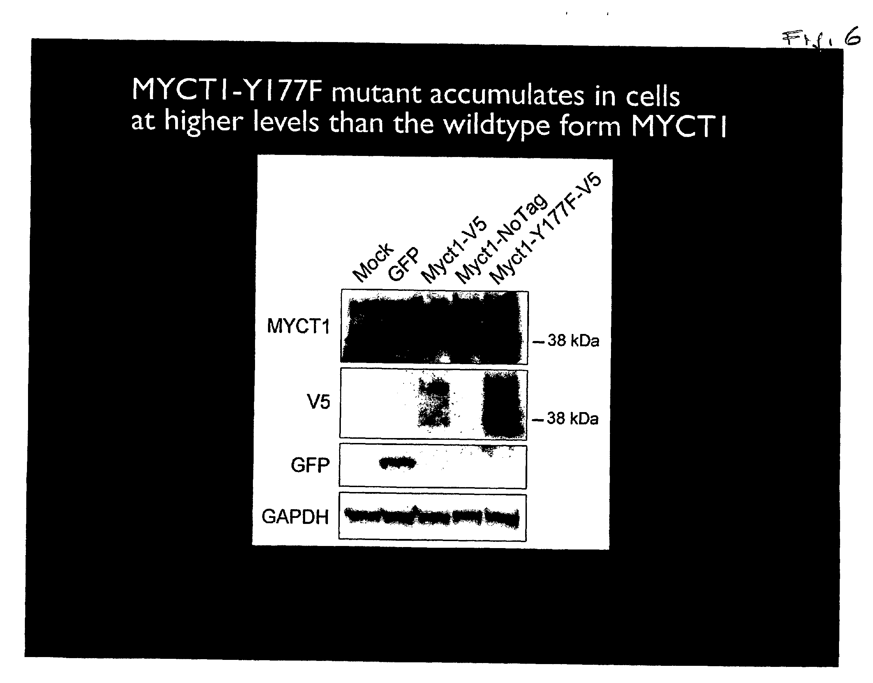

Fig. 2 depicts endothelial gene-I (EGI/MYCT1) and its structure showing N-terminal (N), transmembrane (TM) and C-terminal (C) domain. Two potential isoforms are shown: long MYCT1 (235 aa) and short MYCT1 (187 aa). It is likely that only a short isofom exist, based on the western blot analysis data (see Fig. 6)

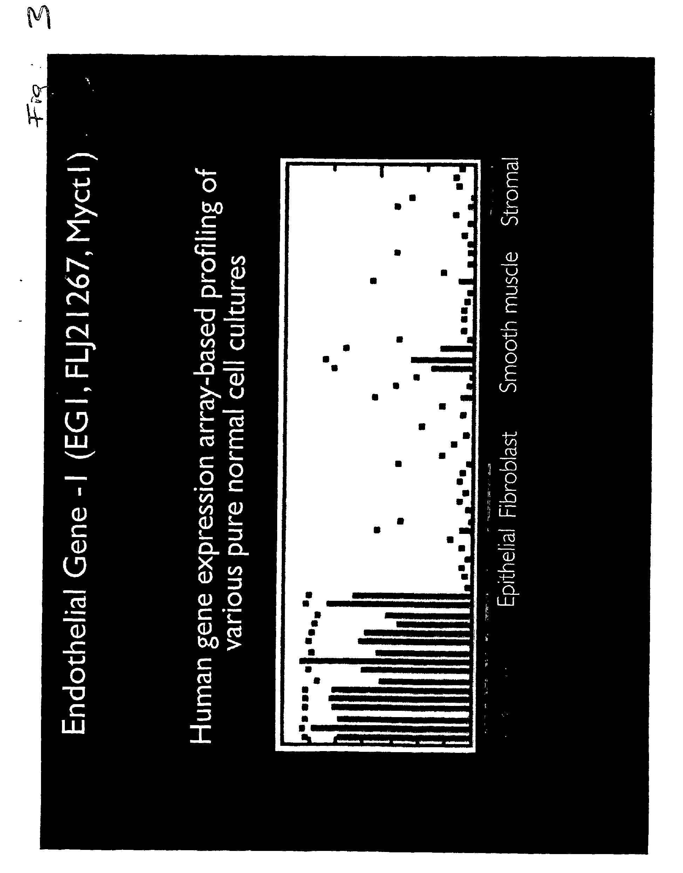

Fig. 3 depicts EGI (MYCT1) gene expression profile based upon: (A) human gene expression array-based profiling of various pure normal cell cultures based on GDS1402 at GEO expression database at NCBI. (B) mouse gene expression profiling of hematopoietic cells based on GDS3997 at GEO expression database at NCBI. The data show high expression of MYCT1 in endothelial cells or in hematopoietic stem and progenitor cells.

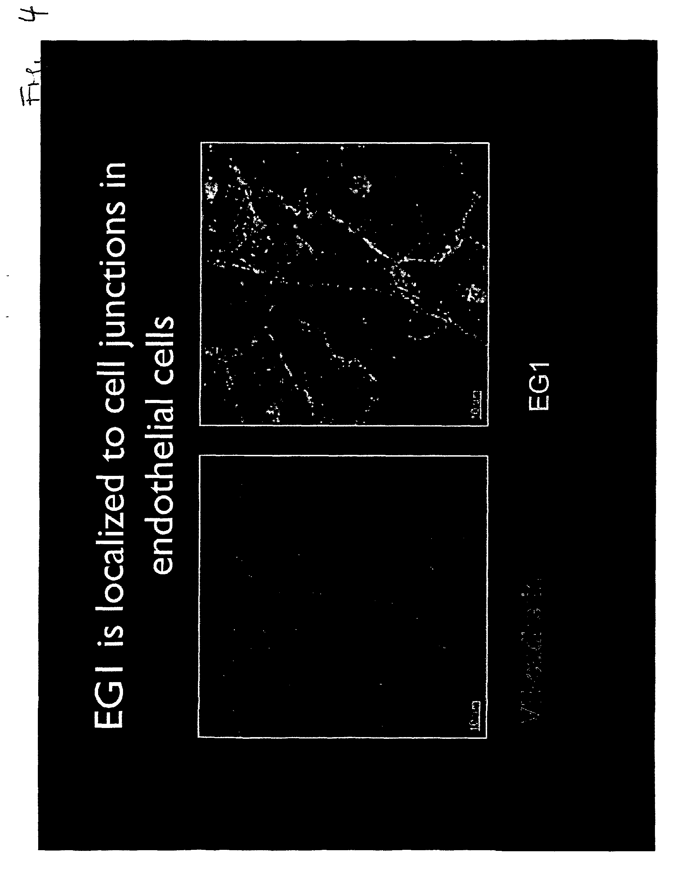

Fig. 4 depicts EGI (MYCT1) cell localization at cell junctions. Endothelial cells were stained for the known junction marker VE-cadherin (left panel) or for Myct1 (right panel). Nuclei are stained with DAPI.

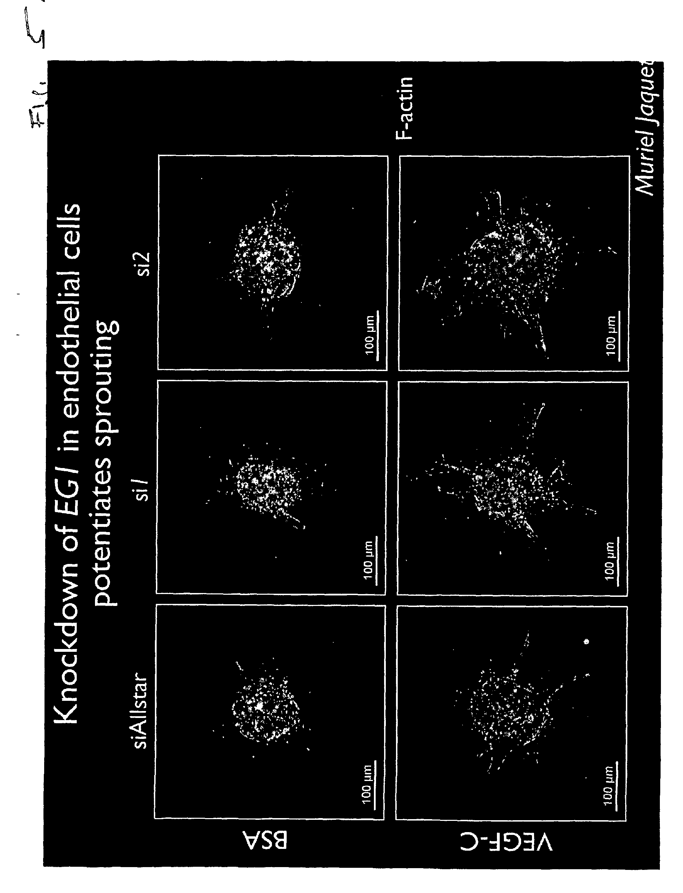

Fig. 5 depicts EGI (MYCT1) knock-out and its influence on sprouting. Human endothelial cells were transfected with the control siRNA, siAllStar, or two different MYCT1 targeting siRNAs (siRNA1 and siRNA2), and the ability of cells to sprout in response to VEGF-C was analysed in comparison to cells treated with the control protein BSA. The data show that sprouting is enhanced in the absence of MYCT1.
Fig. 6 depicts MYCT1-Y177F mutant accumulation in cells as compared to MYCTI in a Western Blot experimentation. Endothelial cells were transduced with the empty control lentivirus and the lentiviruses encoding GFP, sMYCT1, V5-tagged sMYCT1 or V5-tagged sMYCT1 containing a Tyr177 mutated to phenylalanine. The protein was then analysed by Western blotting. The data show greater stability of MYCT1-Tyr177F mutant

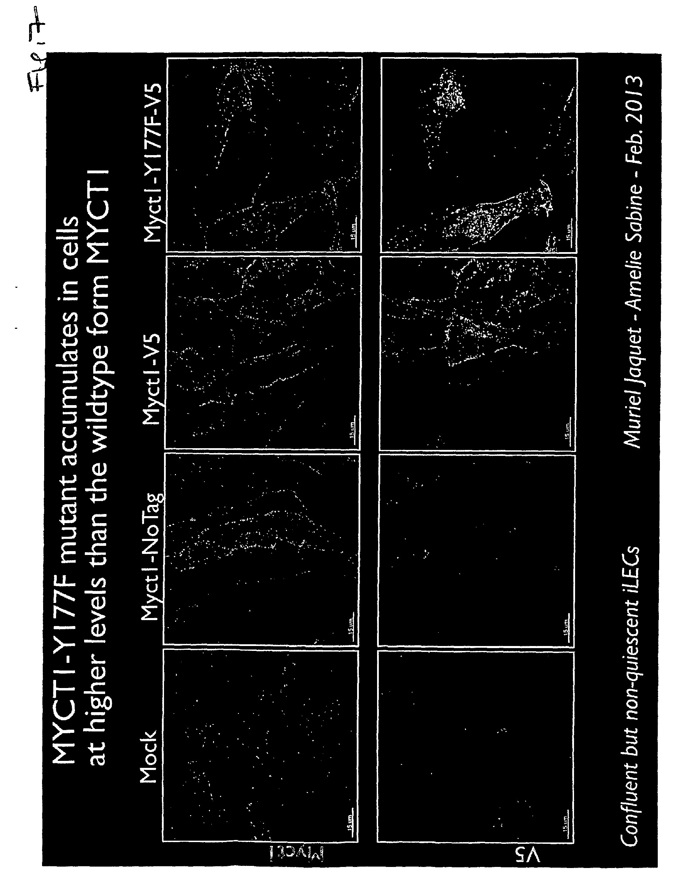

Fig. 7 depicts MYCTI-Y177F mutant accumulation in cells as compared to MYCT1. Endothelial cells, transduced as described in Fig. 6 were stained for MYCT1 or V5 tag.

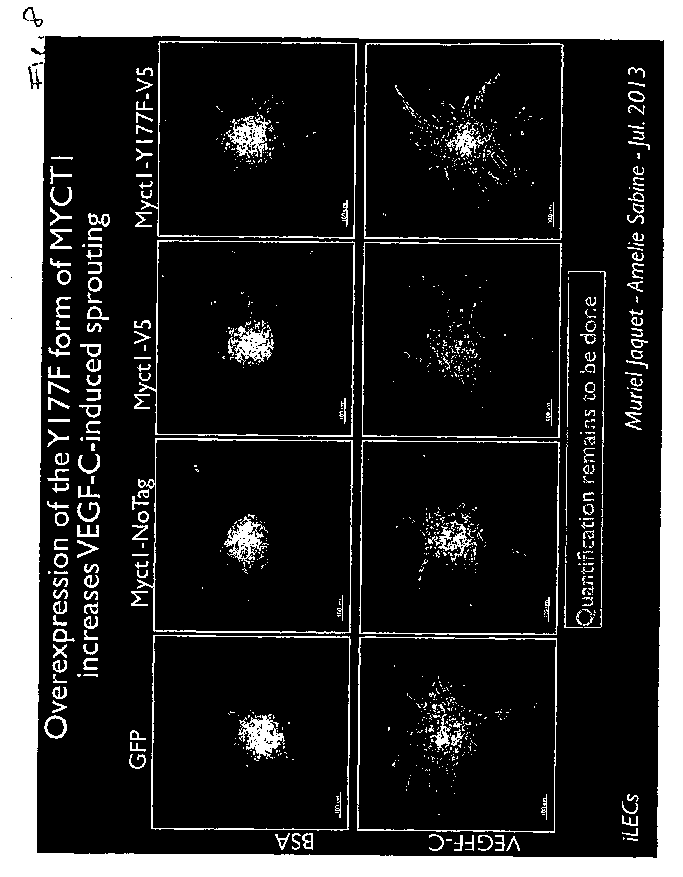

Fig. 8 depicts an over-expression experiment of MYCT1-Y177F in cells resulting in VEGF-C-induced sprouting. Endothelial cells transduced as described in Fig. 6 were analysed for their ability to form sprouts in response to treatment with VEGF-C. The data show that sprouting is enhanced in cells transduced with MYCT1-Tyr177F mutant, suggesting that it may act as an inhibitor of endogenous MYCT1.

### Detailed Description of the Invention

The inventors have identified a novel polypeptide and a nucleic acid encoding same, wherein in a preferred embodiment the polypeptide has a sequence according to SEQ ID NO: 1 (MYCT1).

The polypeptide exhibits preferably in position 177 of its amino acid sequence a tyrosine and more preferably a phenylalanine.

The novel polypeptide and modified polypeptides thereof exhibiting preferably mutations or variations like amino acid replacements, deletions or mutaitons show useful characteristics which may be applied in research and medicine. This novel cell surface endothelial-specific polypeptide or protein can advantageously be used inter alia for targeting and diagnostics in pathological and physiological settings.

MYCT1 according to the invention is a transmembrane protein with large intracellular domain and an extracellular part. Agents targeting MYCT1 may be used for blocking or activating vascular growth or for imaging of MYCT1 expressing vessels in different pathologies, such as cancer, retinal vascular diseases, wound healing, ischemia or myocardial infarctions.

Other embodiments exhibiting advantageous characteristics fall also within the scope of the invention like e.g. the use of truncated versions of the polypeptide according to the invention. One such modified polypeptide is a MYCT1 derivative, which may modulate MYCT1 function, such as MYCT1 intracellular or extracellular domains, or peptides, corresponding to the functional domains of MYCT1. One particularly useful construct will comprise or consist of the or a functional part of the intracellular domain only, herein denoted M1ICD, which will act as an inhibitor of MYCT1 function. This domain also contains in an alternative embodiment a bipartite nuclear localization signal in the intracellular domain RRRRASAPISQWSSSRRSR. Therefore it is possible that such intracellular domain when overexpressed or cleaved from the full-length protein, will be translocated into the nucleus and may play a role in the regulation of transcription. See in this regard also the sequence table below.

In other embodiments the invention relates to a vector comprising a sequence as described above. Such vectors or the polypeptide of the invention may form part or be expressed in a host cell. Such vectors will comprise all necessary elements and sequences for a functional vector known to the skilled person and thus do not need to be described here in further detail.

In another embodiment the invention relates to a method of producing a polypeptide as described above comprising culturing a host cell under suitable conditions and isolating the polypeptide thus produced.

The polypeptide of the invention may be modified as described herein wherein a polypeptide form part of the invention comprising the amino acid sequence of SEQ ID NO: 2 (MYCT1), or a fragment thereof, or a polypeptide having at least 70%, preferably at least 90%, more preferably at least 95% amino acid identity.

In a specific embodiment the polypeptide according to the invention comprises SEQ ID NO: 2. The polypeptide of the invention is preferably of vertebrate origine, more preferably a human, chimpanzee, Rhesus monkey, cow, rat, chicken, zebrafish sequence.

The polypeptide according to the invention comprises preferably a transmembrane region and/or a sequence exhibiting binding to a tyrosine kinase.

The polypeptide according to the invention preferably is endogenously expressed in endothelial cells, preferably in vascular endothelium, or in hematopoietic stem cells

The polypeptide according to the invention can be characterized by variations in the amino acid sequence as well as in amino acid modifications, preferably the amino acid sequence in position 177 contains a phenylalanine or another hydrophobic amino acid, e.g. alanine, valine, leucine, isoleucine, proline, phenylalanine, tryptophane, cysteine and methionine.

The MYCT1 Tyr177Phe polypeptide of the invention will act as dominant-negative inhibitor of endogenous MYCT1 function. In consequence it will be useful as molecule or in a composition in applications wherein EC sprouting is advantageous or shall be induced. Thus, the inventors could provide for a molecule with the MYCT1 Tyr177Phe polypeptide relating to regulation of MYCT1 protein stability and/or its interaction with other partners in functional cascades.

Another preferred embodiment of the invention is a cytoplasmic domain of MYCT1, which exhibits preferably the amino acid modifications as described above. Such a fragment of MYCT1 can also be used and applied as described herein.

Also within the reach of the invention are other modifications of the protein which achieve an interference or stop the interaction with its receptor by way of without limitation e.g. methylation etc.

In another preferred embodiment the amino acid sequence can be modified in order to achieve an inhibitory effect by way of other useful mutations, truncations or deletions within the knowledge of the skilled person.

The polypeptide according to the invention can exhibit biological functions depending on its domains. In a preferred embodiment the polypeptide modulates, preferably inhibits a VEGF or angiopoetins receptors.

The expression pattern of the protein and preferably the modifications of the amino acid sequence provide for a means to target different disorders, diseases by way of inhibition or by way of activation. E.g. the MYCT1 expression in hematopoietic stem cells indicates a use in medical applications wherein such cells will be expanded and applied wherein such multipotent cells are useful. On the other hand the polypeptide or protein of the invention can be used in leukemic diseases wherein the molecule of the invention acts as an inhibitor.

In another embodiment the invention relates to a composition comprising the polypeptide according to the invention and comprises additionally one or more active compounds, carriers, auxiliaries and/or adjuvants.

In another embodiment the polypeptide or the composition according to the invention or a modulator of said polypeptide can be used as a medicament or diagnostic.

In particular the polypeptide and the composition according to the invention or a modulator of said polypeptide can be used for reducing or preventing blood vessel formation, or modulating hematopoietic stem cell functions.

A particular use of the invention relates to preventing, treating or diagnosing cancer, a retinal vascular disease, wound healing, ischemia, myocardial infarction, or atherosclerosis.

Another aspect of the invention is a modulator, preferably an activator or inhibitor, of the polypeptide of the invention.

Also within the scope of the invention is a method for identifying such a modulator with methods generally known in the art.

Such a modulator is selected from the group consisting of a siRNA, a peptide, a protein, a small molecule, zinc finger nuclease, TALEN or CRISPR/CAS9 sequences.

In a specific embodiment the polypeptide according to the invention can be used in a method for detecting, diagnosing a disease or disorder, or for imaging a patient or part of a patient, preferably the vasculature in a patient.

In the following certain terms of the invention will be defined and unless otherwise stated the terms of the invention are to be understood accordingly.

"Nucleic acid" or "polynucleotide" as used herein refers to purine- and pyrimidine-containing polymers of any length, either polyribonucleotides or polydeoxyribonucleotide or mixed polyribo-polydeoxyribonucleotides. This includes single-and double-stranded molecules, i.e., eDNA, mRNA, DNA-DNA, DNA-RNA and RNA-RNA hybrids, as well as "protein nucleic acids" (PNA) formed by conjugating bases to an amino acid backbone. This also includes nucleic acids containing modified bases.

A "nucleic acid molecule" refers to the phosphate ester polymeric form of ribonucleosides (adenosine, guanosine, uridine or cytidine; "RNA molecules") or deoxyribonucleosides (deoxyadenosine, deoxyguanosine, deoxythymidine, or deoxycytidine; "DNA molecules"), or any phosphoester anologs thereof, such as phosphorothioates and thioesters, in either single stranded form, or a double-stranded helix. Double stranded DNA-DNA, DNA-RNA and RNA-RNA helices are possible. The term nucleic acid molecule, and in particular DNA or RNA molecule, refers only to the primary and secondary structure of the molecule, and does not limit it to any particular tertiary forms. Thus, this term includes double-stranded DNA found, inter alia, in linear or circular DNA molecules (e.g., restriction fragments), plasmids, and chromosomes. In discussing the structure of particular double-stranded DNA molecules, sequences may be described herein according to the normal convention of giving only the sequence in the 5' to 3' direction along the nontranscribed strand of DNA (i.e., the strand having a sequence homologous to the mRNA). A "recombinant DNA molecule" is a DNA molecule that has undergone a molecular biological manipulation.

The term "polypeptide" or "protein", in accordance with the present invention, describes linear molecular chains of amino acids, including single chain polypeptides or their fragments, containing more than 5, preferably more than 8, more preferably more than 30 amino acids. The term "fragment" in accordance with the present invention refers to a portion of the polypeptide comprising at least the amino acid residues necessary to maintain the biological activity of the polypeptide. Polypeptides may further form oligomers consisting of at least two identical or different molecules. The corresponding higher order structures of such multimers are, correspondingly, termed homo- or heterodimers, homo- or heterotrimers etc. Furthermore, peptidomimetics of such proteins/polypeptides where amino acid(s) and/or peptide bond(s) have been replaced by functional analogues are also encompassed by the invention. Such functional analogues include all known amino acids other than the 20 gene-encoded amino acids, such as selenocysteine. The terms "polypeptide" and "protein" are used interchangeably herein and also refer to naturally modified polypeptides wherein the modification is effected e.g. by glycosylation, acetylation, phosphorylation and the like. Such modifications are well known in the art.

An "amino acid" in the sense of the invention can be any known naturally or synthetically produced amino acid. The amino acid can be further modified by known techniques including e.g. methylation.

The terms "identical" or percent "identity," or "amino acid identity" in the context of two or more nucleic acids or polypeptide sequences, refer to two or more sequences or subsequences that are the same or have a specified percentage of amino acid residues or nucleotides that are the same (i.e., about 70% identity, preferably 75%, 80%, 85%, 86%, 87%, 88%, 89%, 90%, 91%, 92%, 93%, 94%, 95%, 96%, 97%, 98%, 99%, or higher identity over a specified region (e.g., epitope), when compared and aligned for maximum correspondence over a comparison window or designated region) as measured using e.g. a BLAST sequence comparison algorithms, or by manual alignment and visual inspection. Such sequences are then said to be "substantially identical." The definition also includes sequences that have deletions and/or additions, as well as those that have substitutions. As described below, the preferred algorithms can account for gaps and the like. Preferably, the identity exists over a region that is at least about 25 amino acids or nucleotides in length, or more preferably over a region that is 50-100 amino acids or nucleotides in length, or more preferably over the complete length of said two or more nucleic acids or polypeptide sequences.

In the sense of the invention a "derivative" or "mutant" is characterized by changes in nucleic acid sequence or amino acid sequence wherein single nucleotides or amino acids are changed or modified by insertion, deletion, replacement, or modification well known in the art.

As used herein, the term "fragment" in the context of a proteinaceous agent refers to a peptide or polypeptide comprising an amino acid sequence of at least 2 contiguous amino acid residues, at least 5 contiguous amino acid residues, at least 10 contiguous amino acid residues, at least 15 contiguous amino acid residues, at least 20 contiguous amino acid residues, at least 25 contiguous amino acid residues, at least 40 contiguous amino acid residues, at least 50 contiguous amino acid residues, at least 60 contiguous amino residues, at least 70 contiguous amino acid residues, at least 80 contiguous amino acid residues, at least 90 contiguous amino acid residues, at least 100 contiguous amino acid residues, at least 125 contiguous amino acid residues, at least 150 contiguous amino acid residues, at least 175 contiguous amino acid residues, at least 200 contiguous amino acid residues, at least 250 contiguous amino acid residues, at least 300 contiguous amino acid residues, at least 300 contiguous amino acid residues, at least 500 contiguous amino acid residues, at least 750 contiguous amino acid residues, at least 1000 contiguous amino acid residues, or between 6 to 75 contiguous amino acid residues, between 25 to 150 contiguous amino acid residues, or between 25 to 300 contiguous amino acid residues of the amino acid sequence of a peptide, polypeptide or protein As used herein, the term "fragment" in the context of a nucleic acid refers to a nucleic acid comprising an nucleic acid sequence of at least 2 contiguous nucleotides, at least 5 contiguous nucleotides, at least 10 contiguous nucleotides, at least 15 contiguous nucleotides, at least 20 contiguous nucleotides, at least 25 contiguous nucleotides, at least 30 contiguous nucleotides, at least 35 contiguous nucleotides, at least 40 contiguous nucleotides, at least 50 contiguous nucleotides, at least 60 contiguous nucleotides, at least 70 contiguous nucleotides, at least contiguous 80 nucleotides, at least 90 contiguous nucleotides, at least 100 contiguous nucleotides, at least 125 contiguous nucleotides, at least 150 contiguous nucleotides, at least 175 contiguous nucleotides, at least 200 contiguous nucleotides, at least 250 contiguous nucleotides, or between 8 to 75 contiguous nucleotides, between 25 to 150 contiguous nucleotides, or between 25 to 300 contiguous nucleotides of the nucleic acid sequence encoding a peptide, polypeptide or protein.

A "vector" is any means for the transfer of a nucleic acid into a host cell. A vector may be a replicon to which another DNA segment may be attached so as to bring about the replication of the attached segment. A "replicon" is any genetic element (e.g., plasmid, phage, cosmid, chromosome, virus) that functions as an autonomous unit of DNA replication in vivo, i.e., capable of replication under its own control. The term vector includes both viral and non-viral means for introducing the nucleic acid into a cell in vitro, ex vivo or in vivo. Viral vectors include alphavirus, retrovirus, adena-associated virus, pox, baculovirus, vaccinia, herpes simplex, Epstein-Barr and adenovirus vectors. Non-viral vectors include, but are not limited to plasmids, liposomes, electrically charged lipids (cytofectins), DNA-protein complexes, and biopolymers. In addition to a nucleic acid, a vector may also contain one or more regulatory regions, and/or selectable markers useful in selecting, measuring, and monitoring nucleic acid transfer results (transfer to which tissues, duration of expression, etc.).

A "host cell" in the sense of the invention can be any prokaryotic or eukaryotic cell. Such cells can be applied for polypeptide expression and production of the polypeptides of the invention.

Non-human hosts according to the present invention can be single cells or multi-cellular organisms. Suitable prokaryotic hosts comprise e.g. bacteria of the species Escherichia, Streptomyces, Salmonella or Bacillus. Suitable eukaryotic host cells are e.g. yeasts such as Saccharomyces cerevisiae or Pichia pastoris or chicken cells, such as e.g. DT40 cells. Insect cells suitable for expression are e.g. Drosophila S2 or Spodoptera Sf9 cells. Suitable zebrafish cell lines include, without being limiting, ZFL, SJD or ZF4.

Mammalian host cells that could be used include, human Hela, HEK293, H9 and Jurkat cells, mouse NIH3T3 and C127 cells, COS 1, COS 7 and CV1, quail QC1-3 cells, mouse L cells, mouse sarcoma cells, Bowes melanoma cells and Chinese hamster ovary (CHO) cells. Also within the scope of the present invention are primary mammalian cells or cell lines. Primary cells are cells which are directly obtained from an organism. Suitable primary cells are, e.g., mouse embryonic fibroblasts (MEF), mouse primary hepatocytes, endothelial cells, cardiomyocytes and neuronal cells as well as mouse muscle stem cells (satellite cells) and stable, immortalized cell lines derived thereof. Alternatively, the recombinant protein of the invention can be expressed in stable cell lines that contain the gene construct integrated into a chromosome.

Appropriate culture media and conditions for the above described host cells are known in the art.

For molecular biology techniques, see Sambrook and Russel "Molecular Cloning, A Laboratory Manual", Cold Spring Harbor Laboratory, N.Y. (2001) which is incorporated by reference herewith.

The terms "treating", "treat" and "treatment" include (i) preventing a disease, pathologic or medical condition from occurring (e.g., prophylaxis);(ii) inhibiting the disease, pathologic or medical condition or arresting its development;(iii) relieving the disease, pathologic or medical condition; and/or (iv) diminishing symptoms associated with the disease, pathologic or medical condition. Thus, the terms "treat", "treatment", and "treating" extend to prophylaxis and include prevent, prevention, preventing, lowering, stopping or reversing the progression or severity of the condition or symptoms being treated. As such, the term "treatment" includes medical, veterinary, therapeutic, and/or prophylactic administration, as appropriate.

The term "modulator", "modulating" or "to modulate" in the sense of the invention is to be understood as inhibiting or activating a cellular process or cascade which will lead to an effect in the activity, expression or otherwise in a cell or an organism and which may result in change of the phenotype of a cell or organism or lead to a physiological change or otherwise and which is measurable and/or can be made visible with means generally known in the art.

The terms "inhibit", "inhibiting", and "inhibition" refer to the slowing, halting, or reversing the growth or progression of a disease, infection, condition, or group of cells. The inhibition can be greater than about 20%, 40%, 60%, 80%, 90%, 95%, or 99%, for example, compared to the growth or progression that occurs in the absence of the treatment or contacting. Accordingly and in an equivalent manner the term "activate", "activating", and "activation" is to be understood.

In the sense of the invention "diagnosing" or "diagnostics" is the use of a methodology for the detection of a particular disease or disorder.

The nucleic acid and polypeptide molecules of the invention can be made with known methods of synthesis or recombinant technology well known in the art. Such methods include without limitation chemical synthesis and expression of polypeptides by application of recombinant technologies.

A "composition" according to the invention contains or comprises a polypeptide according to the invention and will be formulated according to the state of the art. Such a composition may contain additional carriers, auxiliaries and/or adjuvants well known in the art.

In the following the invention will be illustrated by way of examples which shall not be construed as limiting in any way but are meant to serve as exemplary embodiments of the invention only.

### Examples

We conducted a bioinformatics analysis of gene expression profiles from 10 most common human tumor types, where we analyzed genes that are highly co-regulated with endothelial markers CDH5 and CD31. We identified several unknown genes encoding potential endothelial-specific proteins. In order to validate these in silico data, we compared our results with other available databases and performed in situ RNA hybridization in mouse embryos. MYCT1 was identified as a new gene whose expression is restricted to endothelial cells in vitro and to the vascular endothelium of capillaries in vivo. SiRNA mediated knock-down approach further revealed that MYCT1 regulates angiogenic sprouting. This is a transmembrane protein, and we propose that MYCT1 may be a target for the development of novel treatment approaches linked to vascular dysfunction, or for diagnostic procedures requiring imaging of vasculature.

### Materials and Methods

### 1. Cell culture

Human endothelial cells were isolated as described (Norrmen et al, 2010). They were cultured on 5 µg/ml fibronectin-coated plates in EBM2 complete medium containing 2% FBS. HeLa cells were cultured in RPMI complete medium containing 10% FBS.

### 2. Cloning of wildtype and mutant MYCT1 lentiviral vectors

*MYCT1* cDNA was amplified using standard molecular biology approaches, and subcloned into pSD44 lentiviral vector, which also encodes a puromycin-resistance gene. Two constructs were generated, with either a C-terminal V5-tagged MYCT1 or a non-tagged form of MYCT1. For the production of the Tyr177Phe mutant, corresponding nucleotides were mutated in a V5-tagged form using PCR-based mutagenesis.

### 3. Lentivirus production and cell transduction

Lentiviruses were produced in 293T cells transfected with 15 µg lentivector, 4 µg pSD16 and 10 µg pSD11 packaging plasmids, which encode for Gag/Pol and Env proteins, respectively. Culture supernatant was collected after 48 hours. Lentiviral particles were quantified using p24 Elisa kit (Gentaur) according to manufacturer's instructions. Cells were transduced at a multiplicity of infection (MOI) of 10 in EBM2 medium with the empty lentivector, a lentivector expressing GFP as a control, or a lentivector expressing MYCT1 wildtype or mutant forms. Three days post-transduction, cells were selected for two days in 300 ng/ml puromycin. MYCT1 exogenous expression was confirmed at the protein level 5 days post-transduction by Western blot analysis and cell immunostaining.

### 4. MYCT1 knockdown by siRNA transfection

For MYCT1 knockdown, cells were transfected with 30-60 nM of the control (Qiagen, AllStars Negative Control siRNA) or *MYCT1* (Qiagen) siRNAs using Lipofectamine RNA1MAX (Invitrogen). *MYCT1* knockdown was confirmed 48 hours post-transfection at the mRNA level by real-time quantitative RT-PCR.

### 5. MYCT1 expression analyses

*MYCT1* expression at the mRNA level was analyzed by real-time quantitative PCR. Total RNA was isolated using Qiagen RNeasy Plus Mini kit (Qiagen). Reverse transcription was performed using Transcriptor First Strand cDNA Synthesis kit (Roche Diagnostics). Real-time qPCR analyses were performed on StepOnePlus (Applied Biosystems), using SYBR Green PCR Master Mix (Applied Biosystems). 18s mRNA was used as an internal control. Sequences of PCR primers were: 18s-Fw (AGGAATTCCCAGTAAGTGCG), 18s-Rev (GCCTCACTAAACCATCCAA), MYCT1-Fw (CTTTCCATCCCTTTCTGCAA) and MYCT1-Rev (GTGCTGATGGTGGGAGAGAT). Analysis of *MYCT1* expression relative to 18s was carried out using the comparative Ct ( Δ Δ Ct) method as described by the manufacturer.

MYCT1 expression at the protein level was analyzed by Western blotting. Cells were lysed in a modified RIPA buffer, containing 50 mM Tris-HCl pH7.4, 0.25 mM Na-deoxycholate, 150 mM NaCl, 2 mM EGTA, 0.1 mM Na₃VO₄, 10 mM NaF, 1 mM PMSF, 1% Triton-X 100 and a complete protease inhibitor mixture (Roche). Protein concentration was measured using BCA kit (Pierce) and samples were resolved by SDS-PAGE, transferred onto Immobilon-P membrane (Millipore), and blotted with mouse anti-V5 (Invitrogen), rabbit anti-human MYCT1 (Abcam), rabbit anti-GFP (Abcam) and rabbit anti-human GAPDH (Sigma). Western blots were developed using the ECL method (SuperSignal West Femto Maximum Sensitivity Substrate; Thermo Fisher Scientific).

### 6. Cell immunostaining

Cells cultured on fibronectin-coated glass coverslips were fixed with 4% PFA, permeabilized with 0.1% Triton X-100 and blocked with 5% donkey serum. Cells were stained with rabbit anti-human MYCT1 (Abcam, ab139945), mouse anti-V5 (Invitrogen) goat anti-mouse VE-cadherin (R&D Systems), and Alexa488-conjugated phalloidin (Invitrogen). Alexa Fluor 488, 555, 594, and 647 fluorochrome-conjugated secondary antibodies (Invitrogen) were used for signal detection.

### 7. Sprouting assay from spheroids

For 3D spheroid sprouting assay, 800 endothelial cells were seeded in round-bottom 96-well plates for spheroid formation as described previously (Korff ana Augustin, 1998). Spheroids were embedded in fibrin gels (2.5 mg/ml fibrinogen, 0.625 U/ml thrombin and 0.15 U/ml aprotinin) and treated with 100 ng/ml BSA (Sigma) or Δ N Δ C VEGF-C for 48 h. Spheroids were fixed in 4% PFA and stained with DAPI and Alexa 488-conjugated phalloidin (Molecular Probes). 10 spheroids were imaged per condition.

**Sequences**

| SEQ ID NO: | Sequence number as contained in the sequence listing | Comment |
|---|---|---|
| 1 | SEQ ID NO: 1 | Human *MYCT1* cDNA |
| 2 | SEQ ID NO: 2 | Human MYCT1 protein (long version NP_079383) |
| 3 | SEQ ID NO: 3 | Human MYCT1 protein (short version - 2nd Met) |
| 4 | SEQ ID NO: 4 | Human mutant *MYCT1-Y177F* CDS |
| 5 | SEQ ID NO: 5 | Human mutant MYCT1-Y177F protein |
| 6 | SEQ ID NO: 6 | MICD Y mutant sequence |
| 7 | SEQ ID NO: 7 | MICD F mutant sequence |

SEQ ID NO: 1
   **Human *MYCT1* cDNA** - **NM_025107** 
ttccttttatgcgaacacaagtatatgaggggttgtgtaaaaattatttttctcttgctgtactacaaagagataga
atcaaactgcttttttcgacatactggtttttctttctgtttttcttctctttcttctatttcttgtggatattatggctaataa
cacaacaagtttagggagtccatggccagaaaacttttgggaggaccttatcatgtccttcactgtatccatg
gcaatcgggctggtacttggaggatttatttgggctgtgttcatttgtctgtctcgaagaagaagagccagtgct
cccatctcacagtggagttcaagcaggagatctaggtcttcttacacccacggcctcaacagaactggatttt
accgccacagtggctgtgaacgtcgaagcaacctcagcctggccagtctcaccttccagcgacaagcttc
cctggaacaagcaaattcctttccaagaaaatcaagtttcagagcttctactttccatccctttctgcaatgtcc
accacttcctgtggaaactgagagtcagctggtgactctcccttcttccaatatctctcccaccatcagcacttc
ccacagtctgagccgtcctgactactggtccagtaacagtcttcgagtgggcctttcaacaccgcccccacct
gcctatgagtccatcatcaaggcattcccagattcctgagtagggtggcttttggtttttgtttctttcttgtcttgtctt
ttattgaaaggaaatcaaaaataggctaaacagaattttgagggcatggcccaaataactcatgagttcca
agttgaaacatggttgtgcaagttggacattacaatgtaaaacacatttcttcaaacacgttttccctttgtttca
aaaaatgtaatatttcccccaagcgttttatatttatgtattttgtattcaatgtgaggcttattaaaaatagtgattc
taatgtaagaatcagctaagatgcattatatatattttaattaaaattaaaacttcagatatttgtggattacaatc
ctcatttacttccaatgtgactaaaaagagaaaaaaaatcactgtgtcactttaaagaaaaatcttctaaggg
atttggattttactttctttagaatgacaagtgaatcatattgacattttacaatcttagattttcttttttttcttttgaga
cagggtcttgatccgtcgcccaggcgggagttgcagtagcatgatcaggactcactgcagcctctatctccc
aggctcaagtaatcctcccatcttagtgccccaagtagctgggactacaggggtgcactaccacaccgggt
tgaattttttttaattttagtagagatgaagtgtcactatgttaccaaggctggtctcaaactcctaaactcagat
gatcctcctgcctcggcctcccaaagtgctggaattagcctggccaatcttggatttttaatggaatatgtgggc
acaaaatgacagaacataggacattctaaagttccttgatttgatcattataagaagtgtgggactcaagca
caggaaactgaactcttttggtgtcattggatgtttcatttttgacactaatttttctggacaaactctttatgtgttttt
cccaagaatagttatctacttcctggaggcaaaatccttggatttactaacatgatgatttaccttttcttcaccgtt
 
gtcgttacattgttagaaaagcaacaggaaaaaatccaattcatttgacctaaaaacaagcctcaagtttaa
aaccaagctcacgtttttcttaagggaaaaattttctttcttaaacttacatctagcaacttggaaagcactttctc
tggggatcttcttttgtaacttgcagacaaataagtatgagtcactggggagagagtttgttattgaaatagat
gttgcccatgaagaattctccttcctggattgactcttaatcatcaggcatcattcctggtttgcttctctacgaatc
tcaattccaacttctctgcagagtctgtacagtgattaagccatgccagatggtctttggtgcacacagttattta
agaatccacttccacaggtggctgcccttgtaaggaagaatgcatccctaaatgtggccaccagagagttc
cagtgggcagatgtctgtggctgcccttctcatttaaggacatgagttcactggagtattactcaaaaagtctgt
ggttcatttccagtattgtgaatatttagtttatgtggccgtttctttgttctttgaacagtgggattttcagtgaaaaa
gtaccctctttttcatttcctattgcagtggtcacagctaatagtgtctgaacatggttcaagaataagagattcc
atgtagcattttctttattattttcatttcccttatattatccatcattccttaaggacaattattcttaataatgcttatag
aaaatgttctctaattaaacatgccaaaaggaaaaagtaagagaaagagggagcaagaagaaaatgg
aagaaaaagggaaaaagctaaccggataaccaatttgttataagttggttttcaacaaagaaatttagca
gccaagtaaggtttcaaggaatattaacttggtatcagggctactttttttttttttttacttgcatgtcatcctta
atgtctaacatgaaaaatcagcaaagagtatggtttttatcaagaatttgtgttgggagtaaaaactgctttata
gctcccaaattaggaagagaagagcagaaatcctctggggcatttaaccatctggcagaattgttgctgca
cccttatcccagttataagacagtcaaaatgactatttcctaaatattgtgagtgtatgaaatgtgaaattaaag
caaaaactggagacttttaatgtatttctttaatttgaaatgttttgtggattgtgaaataaaaataaatttatgtca
agttttattcaaaaaaaaaaaaaaaaaaaaaaaa
SEQ ID NO: 2
   **Human MYCT1 protein (long version NP_079383)** *235 aa* 
MRTQVYEGLCKNYFSLAVLQRDRIKLLFFDILVFLSVFLLFLLFLVDIMANNTT
SLGSPWPENFWEDLIMSFTVSMAIGLVLGGFIWAVFICLSRRRRASAPISQW
SSSRRSRSSYTHGLNRTGFYRHSGCERRSNLSLASLTFQRQASLEQANSFP
RKSSFRASTFHPFLQCPPLPVETESQLVTLPSSNISPTISTSHSLSRPDYWSS
NSLRVGLSTPPPPAYESIIKAFPDS
SEQ ID NO:3
   **Human MYCT1 protein (short version - 2nd Met**) *187 aa* 
MANNTTSLGSPWPENFWEDLIMSFTVSMAIGLVLGGFIWAVFICLSRRRRAS
APISQWSSSRRSRSSYTHGLNRTGFYRHSGCERRSNLSLASLTFQRQASLE
QANSFPRKSSFRASTFHPFLQCPPLPVETESQLVTLPSSNISPTISTSHSLSR
PDYWSSNSLRVGLSTPPPPAYESIIKAFPDS
SEQ ID NO: 4
   Human mutant *MYCT1-Y177F* CDS
   **cloned into pSD44-puro as a C-term V5-tagged construct** 
atggctaataacacaacaagtttagggagtccatggccagaaaacttttgggaggaccttatcatgtccttca
ctgtatccatggcaatcgggctggtacttggaggatttattgggctgtgttcatttgtctgtctcgaagaagaag
agccagtgctcccatctcacagtggagttcaagcaggagatctaggtcttcttacacccacagcctcaacag
aactggatttaccgccacagtggctgtgaacgtcgaagcaacctcagcctggccagtctcaccttccagcg
acaagcttccctggaacaagcaaattcctttccaagaaaatcaagtttcagagcttctactttccatccctttct
gcaatgtccaccacttcctgtggaaactgagagtcagctggtgactctcccttcttccaatatctctcccaccat
cagcacttcccacagtctgagccgtcctgactactggtccagtaacagtcttcgagtgggcctttcaacaccg
cccccacctgcctttgagtccatcatcaaggcattcccagattc
SEQ ID NO: 5
   **Human mutant MYCT1-Y177F protein (also has mutation G71S, which corresponds to the natural variant rs17710008 according to the Uniprot database)** 
MANNTTSLGSPWPENFWEDLIMSFTVSMAIGLVLGGFIWAVFICLSRRRRAS
APISQWSSSRRSRSSYTHSLNRTGFYRHSGCERRSNLSLASLTFQRQASLE
QANSFPRKSSFRASTFHPFLQCPPLPVETESQLVTLPSSNISPTISTSHSLSR
PDYWSSNSLRVGLSTPPPPAFESIIKAFPDS
 Myct1 intracellular domain sequence (MICD). Transmembrane region was identified to be residues 20-42/45 using InterPro program at http://www.ebi.ac.uk/interpro
SEQ ID NO: 6 
ICLSRRRRASAPISQWSSSRRSRSSYTHGLNRTGFYRHSGCERRSNLSLASL
TFQRQASLEQANSFPRKSSFRASTFHPFLQCPPLPVETESQLVTLPSSNISPT
ISTSHSLSRPDYWSSNSLRVGLSTPPPPAYESIIKAFPDS
SEQ ID NO: 7
   MICD mutant sequence

CLSRRRRASAPISQWSSSRRSRSSYTHSLNRTGFYRHSGCERRSNLSLASL
TFQRQASLEQANSFPRKSSFRASTFHPFLQCPPLPVETESQLVTLPSSNISPT
ISTSHSLSRPDYWSSNSLRVGLSTPPPPAFESIIKAFPDS

## Claims

1. A nucleic acid encoding a polypeptide according to SEQ ID NO: 1 (MYCT1).

2. A vector comprising a sequence according to claim 1.

3. A host cell expressing a sequence according to claim 1.

4. A method of producing a polypeptide according to claim 1 comprising culturing the host cell of clam 3 under suitable conditions and isolating the polypeptide produced.

5. A polypeptide comprising the amino acid sequence of SEQ ID NO: 2 (MYCT1), or a fragment thereof, or a polypeptide having at least 70%, preferably at least 90%, more preferably at least 95% amino acid identity.

6. The polypeptide according to claim 5 which comprises SEQ ID NO: 2 and which is a human, chimpanzee, Rhesus monkey, cow, rat, chicken, zebrafish sequence.

7. The polypeptide according to claim 5 or 6 comprising a transmembrane region and/or a sequence exhibiting binding to tyrosine kinases.

8. The polypeptide according to claims 5 to 7 wherein the polypeptide is endogenously expressed in endothelial cells, preferably in vascular endothelium, or in hematopoietic stem cells.

9. The polypeptide according to claim 5 wherein the amino acid sequence in position 177 contains a phenylalanine or another hydrophobic amino acid.

10. The polypeptide according to claim 5 to 9 wherein the protein modulates, preferably inhibits, a VEGF or Angpt receptors.

11. A composition comprising the polypeptide according to claims 3 to 7 additionally one or more carriers, auxiliaries and/or adjuvants.

12. The polypeptide according to claims 5 to 10 or a composition according to claim 11 or a modulator of said polypeptide for use as a medicament.

13. The polypeptide according to claims 5 to 10 or a composition according to claim 11 or a modulator of said polypeptide for use in reducing or preventing blood vessel formation, or modulating hematopoietic stem cell functions, or for use in preventing, treating or diagnosing cancer, a retinal vascular disease, wound healing, ischemia, myocardial infarction, or atherosclerosis.

14. A modulator, preferably an activator or inhibitor, of the polypeptide according to claims 5 to 10, preferably which modulator is selected from the group consisting of a siRNA, a peptide, a protein, a small molecule, zinc finger nuclease, TALEN or CRISPR/CAS9 sequences.

15. The polypeptide according to claims 5 to 10 for use in a method for detecting, diagnosing a disease or disorder, or imaging a patient or part of a patient, preferably the vasculature in a patient.
